# EUROPÄISCHE PATENTANMELDUNG

(11) **EP 3 064 590 A1**
(43) Veröffentlichungstag der Anmeldung: **07.09.2016**
(21) Anmeldenummer: 16000529.4
(22) Anmeldetag: 04.03.2016
(51) Int. Cl.: C12P 41/00, C12P 5/02, C12P 7/06, C12P 7/62

(54) **BIORAFFINERIE-VERFAHREN**

(30) Priorität: 05.03.2015 DE 102015002830
(71) Anmelder: Hochschule Furtwangen, 78120 Furtwangen im Schwarzwald (DE)
(72) Erfinder: Hass, Volker C., 28857 Syke (DE); Oppenländer, Thomas, 78056 Villingen-Schwenningen (DE); Hirschmann, Roland, 78244 Gottmadingen (DE); Reule, Waldemar, 74343 Sachsenheim (DE)
(74) Vertreter: Müller-Boré & Partner Patentanwälte PartG mbB

(57) **Zusammenfassung**

Die vorliegende Erfindung betrifft ein Bioraffinerie-Verfahren, durch das aus Biomasse Methan, Ethanol und eine weitere organische Verbindung gewonnen werden können.

## Beschreibung

Die vorliegende Erfindung betrifft ein Bioraffinerie-Verfahren, durch das aus Biomasse Methan, Ethanol und eine weitere organische Verbindung gewonnen werden können.

Durch biologische Verfahren kann Biomasse zu verschiedenen Produkten verarbeitet werden. Als Biomasse können beispielsweise Nahrungs- und Futtermittel sowie Pflanzen, aber auch biogene organische Reststoffe aus verschiedenen Produktionsprozessen verwendet werden. Als Produkte können unter anderem Energieträger und Kraftstoffe wie Methan und Ethanol oder auch Grund- und Feinchemikalien für die chemische Industrie erhalten werden. Die Produkte werden aus der Biomasse durch verschiedene chemische, beispielsweise enzymatische, Verfahren beziehungsweise durch Fermentation aus dieser unter Bildung eines Reaktionsgemisches erzeugt und durch verfahrenstechnische Prozesse aus dem Reaktionsgemisch aufgearbeitet.

Das Prinzip der Bioraffinerie ist vergleichbar mit dem einer Erdölraffinerie, in welcher der komplex zusammengesetzte Rohstoff Erdöl in einzelne Fraktionen oder Komponenten getrennt wird. Teilweise werden diese durch chemische Verfahren wie beispielsweise das Cracken in hochwertigere Produkt(gemisch)e umgesetzt. Durch Bioraffinerien kann unter anderem Erdöl als wichtiger Rohstoff der chemischen Industrie ergänzt und idealerweise ersetzt werden.

In der jüngeren Vergangenheit wurden verschiedene Bioraffinerie-Verfahren vorgeschlagen. Beispielsweise wird in der Offenlegungsschrift WO 2010/149137 A2 der internationalen Patentanmeldung PCT/DE2010/000705 ein Bioraffinerie-Verfahren vorgeschlagen, mit dem aus Biomasse Chemie-Grundstoffe, Feinchemikalien, Wirkstoffe und speicherbare Energieträger gewonnen werden können.

Bisher wurde jedoch kein Verfahren bereitgestellt, in dem verschiedene Teilverfahren optimal ineinandergreifen, sodass die Nachteile der entsprechenden Einzelverfahren durch synergistische Wechselwirkung ausgeglichen und ihre Vorteile verstärkt werden. Darüber hinaus ist kein Bioraffinerie-Verfahren bekannt, durch das Methan, Ethanol und eine weitere organische Verbindung auf ökoeffiziente und nachhaltige Weise unter optimaler Ausnutzung der Eigenschaften der Teilverfahren erzeugt werden können.

Der vorliegenden Erfindung liegt die Aufgabe zugrunde, ein Bioraffinerie-Verfahren bereitzustellen, mit dem es möglich sein soll, nachhaltig, (öko)effizient, umweltfreundlich und kostengünstig aus Biomasse sowohl Methan und Ethanol als auch weitere Produkte herzustellen.

Diese Aufgabe wird durch die in den Ansprüchen gekennzeichneten Ausführungsformen gelöst.

Insbesondere betrifft die vorliegende Erfindung ein Bioraffinerie-Verfahren, welches die Schritte (S1) des Herstellens einer zuckerhaltigen Lösung und eines feststoffhaltigen Rückstandes aus Biomasse, (S2) des Fermentierens der zuckerhaltigen Lösung mit Hefe unter Bildung von Ethanol und Kohlendioxid und unter Vermehrung der Hefe, (S3) des Abtrennens zumindest eines Teils des gebildeten Ethanols von der vermehrten Hefe, (S4) des Zugebens einer reduzierbaren organischen Verbindung zu zumindest einem Teil der vermehrten Hefe, (S5) des Reduzierens der reduzierbaren organischen Verbindung durch die vermehrte Hefe unter Bildung eines reduzierten Produkts, (S6) des Vergärens der vermehrten Hefe und des feststoffhaltigen Rückstandes unter Bildung von Methan und Kohlendioxid und (S7) des Umsetzens zumindest eines Teils des in den vorstehenden Schritten gebildeten Kohlendioxids mit Wasserstoff zu Methan umfasst.

Die Reihenfolge, in der die Schritte des erfindungsgemäßen Verfahrens durchgeführt werden, unterliegt grundsätzlich keiner besonderen Einschränkung. Die Verfahrensschritte können sowohl sequentiell als auch parallel, gegebenenfalls unter temporärer Speicherung erhaltener Zwischenprodukte, durchgeführt werden. Darüber hinaus können die Schritte des erfindungsgemäßen Verfahrens diskontinuierlich und/oder kontinuierlich betrieben werden. Vorzugsweise werden jedoch alle Schritte des erfindungsgemäßen Bioraffinerie-Verfahrens kontinuierlich betrieben. Nach Bedarf können die Schritte phasenversetzt, das heißt unter Zwischenlagerung/Zwischenspeicherung der Zwischenprodukte, ausgeführt werden.

Grundsätzlich unterliegt die verwendete Biomasse keiner besonderen Einschränkung. Die Biomasse kann tierischen und/oder pflanzlichen Ursprungs sein, wobei sie vorzugsweise pflanzlichen Ursprungs ist. Sie ist vorzugsweise zucker- und/oder stärkehaltig. Neben Stärke können in der Biomasse auch andere biologische Polymere wie Cellulose und/oder Lignocellulose enthalten sein.

Gemäß einer bevorzugten Ausführungsform besteht die Biomasse aus Pflanzen und/oder Pflanzenteilen. Erfindungsgemäß wird besonders bevorzugt Biomasse eingesetzt, die zur herkömmlichen Biogaserzeugung verwendet wird. Dazu zählen insbesondere Klärschlamm, Bioabfall wie Speisereste, Wirtschaftsdünger (Gülle, Mist) und nachwachsende Rohstoffe wie Mais, Gras, Getreide wie Weizen, Roggen, Hafer und Gerste, Futterrübe und Zuckerrübe sowie Silagen daraus.

Verfahren, nach denen aus Biomasse eine zuckerhaltige Lösung und ein feststoffhaltiger Rückstand gewonnen werden können, sind hinlänglich bekannt. So ist es beispielsweise möglich, durch mechanische und/oder thermische Trennverfahren entsprechend geeignete Biomasse in einen flüssigen Anteil, nämlich eine zuckerhaltige Lösung, und einen feststoffhaltigen Rückstand aufzutrennen. Geeignete mechanische Trennverfahren umfassen beispielsweise das Zerkleinern (A) mit Mühlen, Walzen, Schneidwerken oder ähnlichem. Ein geeignetes thermisches/thermochemisches Trennverfahren ist beispielsweise thermochemisches Aufschließen (B) mit Dampf (2), bei dem sich ein Kondensat (4) bildet, welches als Prozesswasser im erfindungsgemäßen Verfahren weiterverwendet oder als Abwasser abgeführt werden kann.

Vor dem Schritt des Herstellens einer zuckerhaltigen Lösung und eines feststoffhaltigen Rückstandes wird die Biomasse vorzugsweise mechanisch zerkleinert (A), beispielsweise durch Häckseln, Schneiden und/oder Mahlen. Darüber hinaus kann die Biomasse vor dem Herstellungsschritt thermisch und/oder chemisch aufgeschlossen werden (B). Ein chemischer Aufschluss kann beispielsweise durch enzymatische Behandlung erfolgen.

Gemäß einer bevorzugten Ausführungsform umfasst der Schritt (S1) des Herstellens der zuckerhaltigen Lösung und des feststoffhaltigen Rückstandes die Schritte (S1-1) des Bereitstellens von Biomasse, (S1-2) des enzymatischen Hydrolysierens der Biomasse unter Bildung eines Hydrolyseproduktes und (S1-3) des Aufteilens des Hydrolyseproduktes in die zuckerhaltige Lösung und den feststoffhaltigen Rückstand.

Enzymatische Hydrolyseverfahren zur Behandlung von Biomasse sind allgemein bekannt. Geeignete Enzyme (5) sind kommerziell erhältlich. In der Biomasse gegebenenfalls vorhandene Kohlenhydrate (Stärke, Cellulose, Hemicellulose) werden durch den Schritt des enzymatischen Hydrolysierens in fermentierbare Zucker überführt, sodass ein Hydrolyseprodukt mit gegenüber der Ausgangsbiomasse erhöhtem Zuckergehalt erhalten werden kann. Darüber hinaus können durch das enzymatische Hydrolysieren aus Proteinen Peptide und Aminosäuren und aus Fetten Fettsäuren und Glycerin gewonnen werden.

Das Aufteilen in die zuckerhaltige Lösung und den feststoffhaltigen Rückstand kann grundsätzlich auf beliebige Weise erfolgen. Beispielsweise kann die zuckerhaltige Lösung durch (Membran)filtrieren, Zentrifugieren oder ähnliche Trennverfahren von dem feststoffhaltigen Rückstand abgetrennt werden.

Weiter umfasst das erfindungsgemäße Verfahren vorzugsweise einen Schritt (S8) des Supplementierens mit essentiellen Nährstoffen, wobei dieser Schritt grundsätzlich an beliebiger Stelle des Verfahrens erfolgen kann, gegebenenfalls auch während einem der anderen Schritte. Vorzugsweise wird vor oder während der Fermentierung (S2) supplementiert. Durch Supplementieren mit, das heißt Zugeben von, essentiellen Nährstoffen kann das Wachstums- und Stoffwechselverhalten von Mikroorganismen in vorteilhafter Weise beeinflusst werden. Dies ist insbesondere dann der Fall, wenn Nährstoffe, die für das Fermentieren notwendig sind, nicht beziehungsweise nicht in ausreichender Menge vorhanden sind. Zu derartigen Mikroorganismen zählen insbesondere die Hefe (Schritte (S2), (S4) und (S5)) sowie auch Methanbakterien, wie sie im Schritt (S6) des Vergärens eingesetzt werden können. Geeignete Nährstoffe zur Supplementierung sind beispielsweise stickstoff- und/oder phosphorhaltige Verbindungen und/oder Vitamine wie beispielsweise Biotin. Vorzugsweise wird vor dem Schritt (S2) des Fermentierens der zuckerhaltigen Lösung diese mit essentiellen Nährstoffen supplementiert. Eine Supplementierung mit Nährstoffen kann aber auch beispielsweise vor den Schritten (S4) (Zugeben einer reduzierbaren organischen Verbindung) oder (S5) (Reduzieren der reduzierbaren organischen Verbindung), gegebenenfalls auch gleichzeitig mit diesen Schritten erfolgen.

Die im Fermentationsschritt verwendete Hefe kann grundsätzlich jede Hefe (Zuckerhefe, *Saccharomyces*) sein, die zur alkoholischen Gärung fähig ist. Beispiele hierfür sind *Saccharomyces bailii, Saccharomyces bayanus, Saccharomyces boulardii, Saccharomyces carlsbergensis,* Backhefe (*Saccharomyces cerevisiae*), *Saccharomyces ellipsoides, Saccharomyces eubayanus, Saccharomyces rouxii.* Es können zugleich mehrere Hefen oder nur eine einzige Hefe verwendet werden. Gemäß einer bevorzugten Ausführungsform ist die Hefe *Saccharomyces cerevisiae.*

Um zu vermeiden, dass das durch die Hefe gebildete Ethanol weiter verstoffwechselt wird, erfolgt der Fermentationsschritt vorzugsweise unter anaeroben Bedingungen, das heißt unter Sauerstoffausschluss. Darüber hinaus wird gemäß der vorliegenden Erfindung in Schritt (S7) das in den Schritten (S2), (S5) und (S6) erzeugte Kohlendioxid mit Wasserstoff zu Methan umgesetzt. Hierbei ist es von Vorteil, wenn das erzeugte Kohlendioxid in möglichst hoher Reinheit, das heißt frei von anderen Gasen wie Sauerstoff vorliegt.

Die Fermentation erfolgt vorzugsweise vollständig, das heißt bis entweder alle vergärbaren Zucker der zuckerhaltigen Lösung fermentiert sind oder die fermentierte Lösung einen Alkoholgehalt erreicht hat, bei dem die Stoffwechselprozesse der Hefe zum Erliegen kommen. Es ist allerdings auch möglich, den Fermentationsschritt vor dem Erreichen eines solchen Zustandes abzubrechen, etwa nach dem Fermentieren von 50 Mol-% der enthaltenen vergärbaren Zucker oder bei Erreichen eines Alkoholgehaltes der fermentierten Lösung von 10 Vol.-%.

Vorzugsweise wird der Fermentationsschritt kontinuierlich betrieben. Es ist aber auch möglich, den Fermentationsschritt und den Schritt des enzymatischen Hydrolysierens der Biomasse gleichzeitig als sogenannten "simultaneous saccharification and fermentation process" (SSF-Prozess) durchzuführen, welcher vorzugsweise diskontinuierlich betrieben wird.

Der Schritt (S3) des Abtrennens zumindest eines Teils des gebildeten Ethanols von der vermehrten Hefe kann auf beliebige Weise und in unterschiedlichen Phasen des erfindungsgemäßen Verfahrens erfolgen. Das Ethanol liegt zunächst als Gemisch mit den weiteren Bestandteilen der fermentierten Lösung vor. Vorzugsweise wird das gebildete Ethanol als ein solches Gemisch mit den weiteren Bestandteilen der fermentierten Lösung weitgehend vollständig, abgesehen von einer von der Hefe gebundenen Restmenge an fermentierter Lösung, vorzugsweise zu mindestens 95 Gew.-%, besonders bevorzugt zu mindestens 99 Gew.-%, von der vermehrten Hefe abgetrennt. Die Abtrennung erfolgt vorzugsweise durch Membranseparation/Membranfiltration. Das heißt, dass die Hefe durch eine Membran zurückgehalten wird. Beispielsweise kann die Membran einen Reaktor, worin die Fermentation durchgeführt wird, in zwei Volumenteile trennen. In diesem Fall wird die fermentierte Lösung so aus dem Reaktor entnommen, dass die vermehrte Hefe von der Membran zurückgehalten wird. Ein derartiger Reaktor wird bevorzugt kontinuierlich betrieben. Des Weiteren kann die Membranseparation wie eine gewöhnliche Filtration erfolgen, wobei die Membran als Filtermedium verwendet wird.

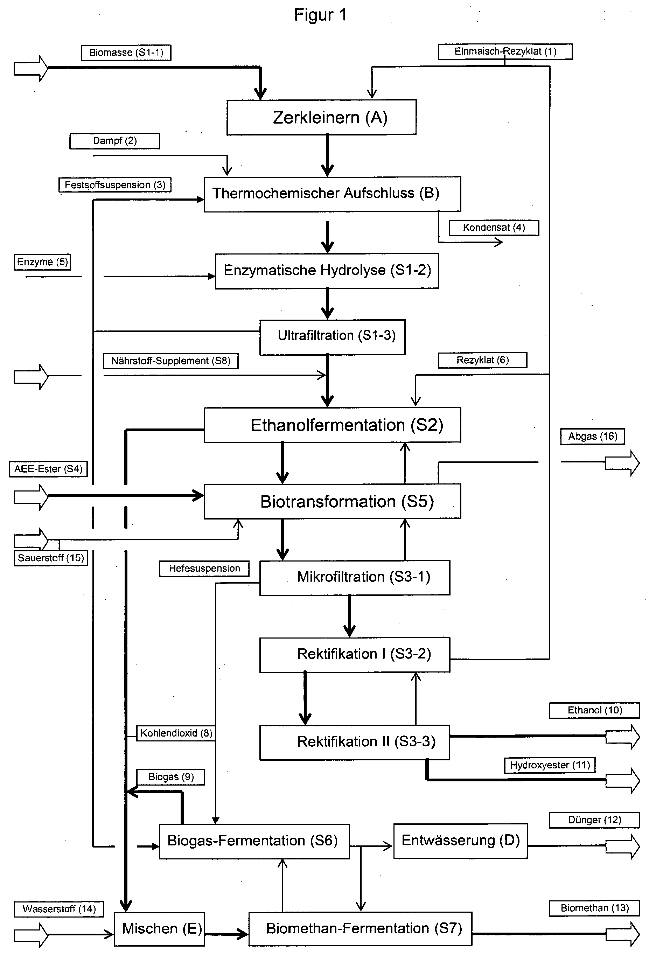

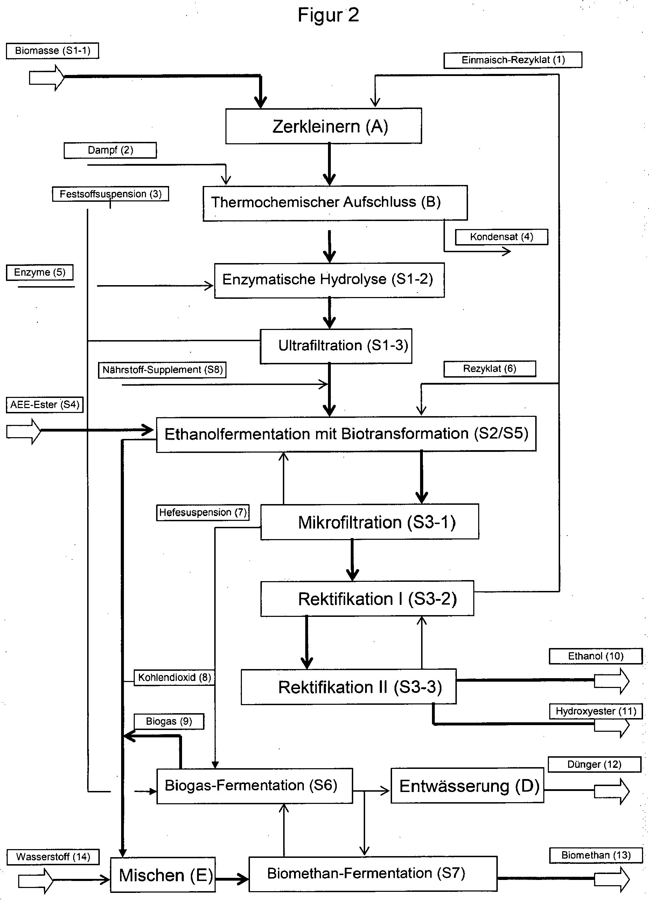

Das Ethanol in der abgetrennten fermentierten Lösung wird vorzugsweise durch Rektifikation aufgereinigt (Schritt(e) (S3-2) bzw. (S3-3)). Wie in Figuren 1 und 2 dargestellt, ist die Rektifikation vorzugsweise zweistufig, das heißt, das im Kopfprodukt der ersten Rektifikationsstufe enthaltene Ethanol wird in der zweiten Rektifikationsstufe weiter aufkonzentriert, gegebenenfalls mit Absolutierung. Gemäß einer weiteren Ausführungsform des erfindungsgemäßen Verfahrens ist die erste Rektifikationsstufe mit dem Fermentationsschritt gekoppelt. Ein geeignetes Rektifikationsverfahren, worin erste und zweite Reaktionsstufen gekoppelt sind, ist beispielsweise das Biostil-Verfahren.

Gemäß einer weiteren Ausführungsform erfolgt der Schritt des Abtrennens zumindest eines Teils des Ethanols (S3) nach den Schritten (S2) und (S5), wie beispielsweise in Figuren 1 und 2 dargestellt. In diesem Fall kann in Schritt (S3) sowohl das in Schritt (S2) gebildete Ethanol als auch das in Schritt (S5) aus der reduzierbaren organischen Verbindung erhaltene reduzierte Produkt (beispielsweise ein aus einem Ketoester erhaltener Hydroxyester) von der Hefe abgetrennt und isoliert werden. Wie vorstehend erläutert, erfolgt diese Abtrennung vorzugsweise durch Membranfiltration (S3-1). Die weitere Aufreinigung/Auftrennung von Ethanol (10) und reduziertem Produkt (11) erfolgt vorzugsweise destillativ, wie vorstehend erläutert.

Der feste Anteil des Sumpfproduktes der Rektifikation (Schlempe) wird vorzugsweise als Einmaisch-Rezyklat (1) im Schritt des Herstellens einer zuckerhaltigen Lösung und eines feststoffhaltigen Rückstandes aus Biomasse verwendet. Dadurch kann die Menge des anfallenden Abwassers erheblich gesenkt werden, was die Umweltfreundlichkeit des erfindungsgemäßen Verfahrens weiter steigert. Der flüssige Anteil des Sumpfproduktes der Rektifikation umfasst vorwiegend Wasser und wird vorzugsweise als Rezyklat (6) Schritt (S5) zugeführt.

Die im Schritt (S4) zuzugebende reduzierbare organische Verbindung unterliegt keinerlei Einschränkung. Es kann jedwede reduzierbare organische Verbindung eingesetzt werden, vorausgesetzt, dass sie unter Verwendung der in der Fermentation (S2) vermehrten Hefe als Biokatalysator reduziert werden kann. Als reduzierbare organische Verbindung können beispielsweise Carbonylverbindungen wie Aldehyde und/oder Ketone bzw. deren Gemische zugegeben werden, um diese beispielsweise zu entsprechenden primären und/oder sekundären Alkoholen zu reduzieren. Vorzugsweise wird jedoch nur ein einziger Aldehyd oder ein einziges Keton zugegeben, welches im dem Schritt der Biotransformation (S5) zu dem jeweiligen reduzierten Produkt überführt wird.

Besonders bevorzugt wird eine prochirale reduzierbare organische Verbindung. Die Reduktion einer entsprechenden prochiralen funktionellen Gruppe durch die vermehrte Hefe erfolgt enantioselektiv. Das heißt, in dem Schritt (S5) des Reduzierens/Umsetzens einer derartigen prochiralen reduzierbaren organischen Verbindung wird vorzugsweise ein weitgehend enantiomerenreines reduziertes Produkt erhalten. Gemäß einer weiteren Ausführungsform des erfindungsgemäßen Verfahrens wird in Schritt (S4) des Zugebens einer reduzierbaren organischen Verbindung ein prochirales Keton zugegeben, welches in Schritt (S5) zu einem primären und/oder sekundären, weitgehend enantiomerenreinen Alkohol als reduziertes Produkt umgesetzt wird.

Gemäß einer besonders bevorzugten Ausführungsform der vorliegenden Erfindung wird als prochirales Keton ein prochiraler Ketoester verwendet, der als reduzierbare organische Verbindung in Schritt (S5) enantioselektiv zu einem entsprechenden chiralen primären und/oder sekundären Alkohol umgesetzt wird. Besonders bevorzugt wird als Keton ein β-Ketoester eingesetzt, insbesondere Ethylacetoacetat ("EAA"), welches prochiral ist. Dieses wird vorwiegend enantiomerenrein zu Ethyl-(S)-3-hydroxybutyrat ("E3HB") reduziert/umgesetzt. Gemäß dieser Ausführungsform kann kostengünstig und effizient weitgehend enantiomerenreines Ethyl-(S)-3-hydroxybutyrat hergestellt werden.

"Weitgehend enantiomerenrein" bedeutet hierbei, dass der chirale Alkohol mit einem Enantiomerenüberschuss (enantiomeric excess, abgekürzt "ee") von mindestens 90 %, vorzugsweise mindestens 95 %, besonders bevorzugt mindestens 99 %, erhalten wird. Gemäß einer weiteren Ausführungsform des erfindungsgemäßen Verfahrens wird der Schritt (S5) unter anaeroben Bedingungen durchgeführt. Wenn die Reduktion in Schritt (S5) des Umsetzens einer prochiralen Verbindung, beispielsweise eines prochiralen Ketons, unter anaeroben Bedingungen erfolgt, kann ein besonders hoher Enantiomerenüberschuss erzielt werden. Darüber hinaus kann die Enantiomerenreinheit auch mit weiteren Maßnahmen, wie beispielsweise der Zugabe von weiteren Substraten (z.B. Allylalkohol, D-Gluconolacton und Methylvinylketon), als auch über die Fahrweise des Verfahrens, beeinflusst werden.

Vorzugsweise wird - zusätzlich zu der reduzierbaren organischen Verbindung - ein von der Hefe vergärbarer Zucker zugegeben. Der Zucker kann beispielsweise in Form der zuckerhaltigen Lösung zugegeben werden, es ist aber auch möglich, Zucker aus einer anderen Quelle zuzugeben. Alternativ oder ergänzend kann ein Alkohol, beispielsweise Ethanol, zugegeben werden. Der zugegebene Zucker und/oder der zugegebene Alkohol dienen als Energiequelle der Hefe. Sofern Ethanol als Alkohol zugegeben wird, wird der Schritt des Umsetzens vorzugsweise unter aeroben Bedingungen durchgeführt, da die Hefe ansonsten das Ethanol nicht verstoffwechseln kann. Erfindungsgemäß ist es möglich, das Ethanol in diesem Schritt separat zuzugeben, es ist aber auch möglich, das Ethanol (und den Zucker) durch nur teilweises Abtrennen der fermentierten Lösung von der Hefe zuzugeben.

Enantiomerenreine sekundäre Alkohole sind hochwertige Zwischen- und Endprodukte, die in einer Vielzahl von Anwendungen eingesetzt werden. Nachdem die Trennung von Enantiomeren besonders aufwändig ist, stellt es einen besonderen Vorteil dar, wenn diese bereits im zugrundeliegenden Herstellungsverfahren weitgehend enantiomerenrein hergestellt werden können.

Der Schritt (S6) des Vergärens der vermehrten Hefe und des feststoffhaltigen Rückstandes unter Bildung von Methan kann sowohl einstufig als auch zweistufig erfolgen. In der zweistufigen Variante erfolgt zunächst ein Versäuerungsschritt gefolgt von einem Methanerzeugungsschritt. Für den Vergärungsschritt sind grundsätzlich alle bekannten Verfahren zum Erzeugen von Biogas geeignet. Vorzugsweise wird der feststoffhaltige Rückstand und/oder die vermehrte Hefe in Form einer Feststoffsuspension (3) bzw. (7) dem Schritt (S6) zugeführt.

An den Schritt (S6) kann sich, nach Abtrennen des Produktgases, ein Entwässerungsschritt (D) anschließen. Das erhaltene Wasser kann als Prozesswasser im erfindungsgemäßen Verfahren weiterverwendet werden, oder ganz oder teilweise als Abwasser abgeführt werden. Der nach dem Entwässern (D) verbleibende Rückstand kann als Dünger (12) verwendet werden.

Das erfindungsgemäße Bioraffinerie-Verfahren umfasst weiter einen Schritt (S7) des Umsetzens zumindest eines Teils des in den vorstehenden Schritten gebildeten Kohlendioxids mit Wasserstoff zu Methan (Methanisierungsschritt). Kohlendioxid entsteht insbesondere im Fermentationsschritt (S2) (Erzeugung von Ethanol), im Schritt des Umsetzens eines Aldehyds und/oder Ketons (S5) sowie im Vergärungsschritt (S6) (Biogaserzeugung), kann aber auch in anderen Schritten anfallen.

Vorzugsweise wird das in den vorgenannten Schritten (insbesondere (S2), (S5) und/oder (S6)) anfallende Kohlendioxid (8) mit dem im Vergärungsschritt erzeugten Biogas (9) vermengt. Es ist für die folgende Methanisierung (im Gegensatz zu konventionellen thermochemischen Methanisierungsverfahren) nicht notwendig, das anfallende Kohlendioxid vor dem Zumischen zu trocknen/entwässern. Das erhaltene Biogas wird mit Wasserstoff (14) gemischt (E) und kann direkt ohne weitere Vorbehandlung für den Methanisierungsschritt (S7) verwendet werden. Vorzugsweise wird der Wasserstoff stöchiometrisch (0,9 bis 1,1 Äquivalente, vorzugsweise 1 Äquivalent; dabei bedeutet 1 Äquivalent vier Mol Wasserstoff pro einem Mol Kohlendioxid) zugegeben, entsprechend der folgenden Reaktionsgleichung (Sabatier-Reaktion):

CO₂ + 4H₂ → CH₄ + 2H₂O

Unter Umständen kann es jedoch erforderlich sein, den Wasserstoff überstöchiometrisch (mehr als 1 Äquivalent) zuzugeben. Durch eine überstöchiometrische Fahrweise kann die Restkonzentration von Kohlendioxid im erhaltenen Produkt (Biomethan) gesenkt werden.

Für die Methanisierung von Kohlendioxid wurden in der Vergangenheit eine Vielzahl verschiedener Verfahren vorgeschlagen, welche in der vorliegenden Erfindung zum Einsatz kommen können. Beispielsweise kann die Methanisierung von Kohlendioxid mittels eines thermochemischen Katalyseverfahrens erfolgen.

Vorzugsweise erfolgt der Methanisierungsschritt (S7) gemäß den in den Offenlegungsschriften der deutschen Patentanmeldungen DE 10 2011 051 836 beziehungsweise DE 10 2013 001 689 vorgeschlagenen biologischen Verfahren. Im letztgenannten Verfahren erfolgt die vorstehende Sabatier-Reaktion katalysiert durch dieselben Bakterien, die im Vergärungsschritt zur Biogaserzeugung eingesetzt werden. In jedem der genannten Verfahren wird das Biogas-Produkt aus dem Vergärungsschritt (S6) ohne weitere vorherige Reinigung mit dem Wasserstoff stöchiometrisch oder überstöchiometrisch vermischt, einem Bio-Methanisierungs-Reaktor zugeführt, der Bio-Methanisierungs-Reaktor mit Bakteriensuspension beispielsweise aus dem Vergärungsschritt beschickt und das Kohlendioxid mit dem Wasserstoff von den Methanbakterien der Suspension zu Methan und Wasser umgesetzt. Die Suspensionsphase wird bevorzugt im Gegenstrom oder im Gleichstrom zur Gasphase geführt. Weitere bevorzugte Ausgestaltungen dieses Verfahrens können der vorstehend genannten Offenlegungsschriften DE 10 2011 051 836 A1 und DE 10 2013 001 689 A1 entnommen werden.

Ein Vorteil dieser Ausführungsform besteht darin, dass der Methangehalt des Biogases (13) deutlich erhöht wird. So können aufwändige Schritte zur Abtrennung des Kohlendioxids gegebenenfalls entfallen, was sich positiv auf die Verfahrenskosten auswirkt. Darüber hinaus kann durch diese Ausführungsform ein kohlendioxidneutrales Bioraffinerie-Verfahren, beziehungsweise ein Bioraffinerie-Verfahren, bei dem kein Kohlendioxid anfällt, bereitgestellt werden.

Gemäß einer bevorzugten Ausführungsform der vorliegenden Erfindung wird der im Methanisierungsschritt (S7) eingesetzte Wasserstoff (14) durch Elektrolyse von Wasser mit elektrischem Strom aus einer oder mehreren regenerativen Energiequelle(n) und/oder mit Überschussstrom aus dem elektrischen Stromnetz gewonnen. Überschussstrom ist besonders kostengünstig. Daher wirkt sich die Erzeugung von Wasserstoff aus Überschussstrom besonders vorteilhaft auf die Verfahrenskosten aus. Strom aus regenerativen Energiequellen ist besonders umweltfreundlich. Darüber hinaus fällt bei seiner Erzeugung kein Kohlendioxid an. Dadurch kann der Einsatz von Überschussstrom dazu beitragen, das erfindungsgemäße Verfahren kohlendioxidneutral zu gestalten bzw. so einzurichten, dass kein Kohlendioxid anfällt. Wird mit elektrischem Strom aus den vorstehend genannten Energiequellen elektrolytisch Wasserstoffgas gewonnen, so wirkt sich dies also vorteilhaft auf die Kosten- und/oder Kohlendioxidbilanz des erfindungsgemäßen Verfahrens aus.

Gemäß einer bevorzugten Ausführungsform ist das erfindungsgemäße Bioraffinerie-Verfahren kohlendioxidneutral und/oder energieneutral oder sogar kohlendioxidnegativ und/oder energiepositiv. Das heißt, dass durch das Verfahren nicht mehr Kohlendioxid aus nicht-regenerativen Energiequellen freigesetzt wird, als Kohlendioxid gebunden wird, beziehungsweise dass mindestens so viel Energie erzeugt wird, wie Energie benötigt wird.

Gemäß einer weiteren bevorzugten Ausführungsform fällt in dem erfindungsgemäßen Verfahren kein Kohlendioxid an. Das heißt, Kohlendioxid wird weder als Produkt, Nebenprodukt oder Emission erhalten. Dies schließt nicht aus, dass Kohlendioxid gebildet wird, sofern es im erfindungsgemäßen Verfahren weiter umgesetzt wird, beispielsweise zu Methan. Die vorstehende Ausführungsform kann insbesondere durch Schritt (S7), nämlich die in das Verfahren integrierte Konversion (Umsetzung) von als Zwischenpropdukt entstehendem Kohlendioxid zu Methan realisiert werden.
Figur 1 zeigt schematisch eine bevorzugte Ausführungsform des erfindungsgemäßen Verfahrens. Ethanolfermentation und Biotransformation werden voneinander getrennt durchgeführt. Als Energiequelle für die Hefe in Schritt (S5) dient vorwiegend das in Schritt (S2) gebildete Ethanol. Schritt (S5) wird unter aeroben Bedingungen durchgeführt. Sauerstoff (15) kann beispielsweise durch Luftzufuhr bereitgestellt werden.
Figur 2 zeigt schematisch eine weitere bevorzugte Ausführungsform des erfindungsgemäßen Verfahrens. Ethanolfermentation und Biotransformation werden in einer Stufe gemeinsam durchgeführt. Als Energiequelle für die Hefe in Schritt (S5) dient vorwiegend der in Schritt (S1), insbesondere Schritt (S1-2), gebildete Zucker. Schritt (S5) wird unter anaeroben Bedingungen durchgeführt. Daher wird bei der Biotransformationsreaktion neben der Reduktion der reduzierbaren organischen Verbindung überwiegend Zucker (beispielsweise zu Ethanol) umgesetzt.

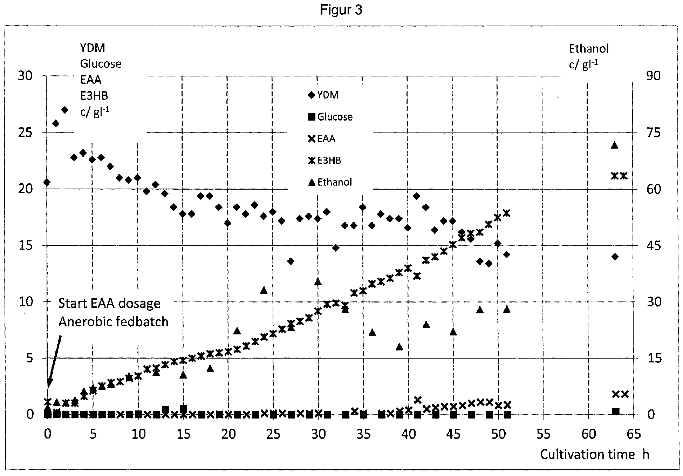

Figur 3 zeigt die jeweilige Konzentration von Hefetrockensustanz ("yeast dry matter";
YDM), Glucose, Ethylacetoacetat (EAA), Ethyl-(S)-3-hydroxybutyrat (E3HB) und Ethanol des nachstehenden Beispiels in Gramm pro Liter (gl⁻¹) in Abhängigkeit der Kultivierungszeit ("Cultivation time") in Stunden.
Figur 4 zeigt das Verfahrensschema mit Prozess-Stellgrößen, welche dem nachstehenden Beispiel zugrunde liegen.

Das nachstehende Beispiel dient als weitere Erläuterung der vorliegenden Erfindung, ohne darauf beschränkt zu sein.

### Beispiel: Anareobe Biokatalyse von EAA zu E3HB

### Verwendetes Verfahren

Die Versuche wurden in einem 5 Liter Bioreaktor im Fedbatch Betrieb durchgeführt. Dazu kamen folgende Geräte zum Einsatz (siehe Tabelle 1):

**Tabelle 1: Materialien**

| |
|---|
| Bioreaktor Bioflo 3000 New Brunswik 5 L |
| Dosierwaagen Sartorius LA 34001 P |
| Gasuhr Ritter |

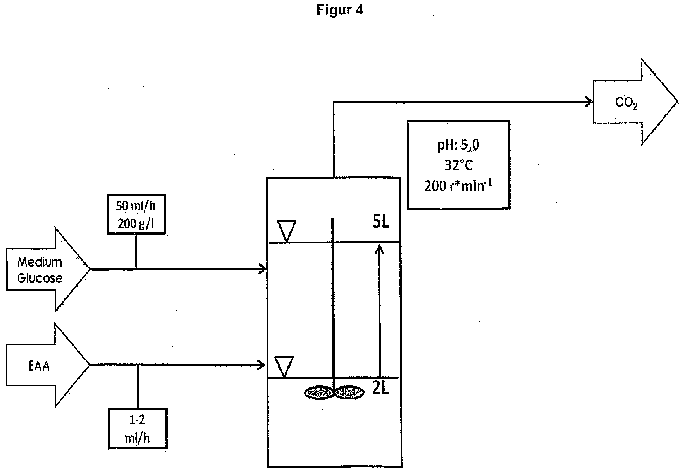

Die wichtigsten Stellgrößen für die Versuchsdurchführung können aus Figur 4 entnommen werden. Das Medium für die Biokatalyse mit *Saccharomyces cerevisae* hatte folgende Zusammensetzung (siehe Tabelle 2):

**Tabelle 2: Medium für die Biokatalyse**

| | pro Liter | |
|---|---|---|
| Glucose-Monohydrat | 220,0 | g |
| Magnesiumsulfat-Heptahydrat | 10,3 | g |
| Natriumchlorid | 5,0 | g |
| Phosphorsäure 85 % | 6,8 | ml |
| Schwefelsäure 95 % | 19,5 | ml |
| Kalilauge 10 mol/L | 3,9 | ml |
| Ammoniakwasser 25 % | 73,3 | ml |
| Vitaminlösung | 2,0 | ml |
| Spurenelemente-Lösung | 2,0 | ml |

Für die Analyse der Prozessparameter kamen folgende Analysemethoden zum Einsatz (siehe Tabelle 3):

**Tabelle 3: Analysemethoden**

| parameter | Methode | Hersteller | Typ |
|---|---|---|---|
| Glucose | enzyme kit photometer | r-biopharm | 10716251035 UV-mini 1240 |
| Biomasse (YDM) | Moisture analyzer | sartorius | MA45 |
| Ethanol | enzyme kit photometer | r-biopharm | 10176290035 UV-mini 1240 |
| EAA | HPLC | Dionex | Ultimate 3000 PDA 100 |
| E3HB | HPLC | Dionex | Ultimate 3000 PDA 100 |

Die HPLC wurde wie folgt betrieben (siehe Tabelle 4):

**Tabelle 4: Betriebsparameter HPLC**

| **HPLC (Dionex)** | |
|---|---|
| Column | Rezex ROA-Organic Acid (8%) Phenomenex |
| Dimension | 300 x 7.8 mm |
| Mobile phase | 0.005 N H₂SO₄ |
| Flow rate | 0.3 ml*min⁻¹ |
| Detector | UV @ 210 nm |
| Temperature | 25 °C |
| Retention time E3HB | 47,84 min |
| Retention time EAA | 58,56 min |

Durch die vorliegende Erfindung wird ein Bioraffinerie-Verfahren bereitgestellt, mit dem es möglich ist, effizient, umweltfreundlich und kostengünstig aus Biomasse neben Methan und Ethanol eine weitere organische Verbindung herzustellen. Diese weitere organische Verbindung kann ein hochwertiges chemisches Erzeugnis, beispielsweise ein weitgehend enantiomerenreiner chiraler primärer oder sekundärer Alkohol und insbesondere ein (weitgehend enantiomerenreiner) Hydroxyester sein. Damit können durch das erfindungsgemäße Verfahren nicht nur Methan und Ethanol, sondern auch hochwertige chemische Erzeugnisse auf umweltschonende Weise aus Biomasse erzeugt werden.

## Patentansprüche

1. Bioraffinerie-Verfahren, welches die folgenden Schritte umfasst:
(S1) Herstellen einer zuckerhaltigen Lösung und eines feststoffhaltigen Rückstandes aus Biomasse,
(S2) Fermentieren der zuckerhaltigen Lösung mit Hefe unter Bildung von Ethanol und Kohlendioxid und unter Vermehrung der Hefe,
(S3) Abtrennen zumindest eines Teils des gebildeten Ethanols von der vermehrten Hefe,
(S4) Zugeben einer reduzierbaren organischen Verbindung zu zumindest einem Teil der vermehrten Hefe,
(S5) Reduzieren der reduzierbaren organischen Verbindung durch die vermehrte Hefe unter Bildung eines reduzierten Produkts,
(S6) Vergären der vermehrten Hefe und des feststoffhaltigen Rückstandes unter Bildung von Methan und Kohlendioxid und
(S7) Umsetzen zumindest eines Teils des in den vorstehenden Schritten gebildeten Kohlendioxids mit Wasserstoff zu Methan.

2. Bioraffinerie-Verfahren nach Anspruch 1, welches weiter den folgenden Schritt umfasst:
(S8) Supplementieren mit essentiellen Nährstoffen.

3. Bioraffinerie-Verfahren nach Anspruch 1 oder 2,
wobei der Wasserstoff durch Elektrolyse von Wasser mit elektrischem Strom aus einer oder mehreren regenerativen Energiequelle(n) und/oder mit Überschussstrom aus dem elektrischen Stromnetz gewonnen wird.

4. Bioraffinerie-Verfahren nach einem der Ansprüche 1 bis 3,
wobei der Schritt (S1) des Herstellens der zuckerhaltigen Lösung und des feststoffhaltigen Rückstandes die folgenden Schritte umfasst:
(S1-1) Bereitstellen von Biomasse,
(S1-2) enzymatisches Hydrolysieren der Biomasse unter Bildung eines Hydrolyseproduktes und
(S1-3) Aufteilen des Hydrolyseproduktes in die zuckerhaltige Lösung und den feststoffhaltigen Rückstand.

5. Bioraffinerie-Verfahren nach einem der Ansprüche 1 bis 4,
wobei der Schritt des Reduzierens der reduzierbaren organischen Verbindung (S5) unter anaeroben Bedingungen erfolgt.

6. Bioraffinerie-Verfahren nach einem der Ansprüche 1 bis 5,
wobei in dem Schritt (S4) des Zugebens einer reduzierbaren organischen Verbindung ein Aldehyd und/oder ein Keton zugegeben wird, welches in dem Schritt (S5) zu einem primären und/oder sekundären Alkohol als reduziertes Produkt umgesetzt wird.

7. Bioraffinerie-Verfahren nach einem der Ansprüche 1 bis 6,
wobei in dem Schritt (S4) des Zugebens einer reduzierbaren organischen Verbindung ein prochirales Keton zugegeben wird, welches in dem Schritt (S5) zu einem weitgehend enantiomerenreinen sekundären Alkohol als reduziertes Produkt umgesetzt wird.

8. Bioraffinerie-Verfahren nach Anspruch 7,
wobei das Keton Ethylacetoacetat ist und der sekundäre Alkohol Ethyl-(S)-3-hydroxybutyrat ist.

9. Bioraffinerie-Verfahren nach einem der Ansprüche 1 bis 8,
wobei die Biomasse aus Pflanzen und/oder Pflanzenteilen besteht.

10. Bioraffinerie-Verfahren nach einem der Ansprüche 1 bis 9,
welches kohlendioxidneutral und/oder energieneutral ist.

11. Bioraffinerie-Verfahren nach einem der Ansprüche 1 bis 10,
worin kein Kohlendioxid anfällt.
